# EUROPÄISCHE PATENTSCHRIFT

(11) **EP 0 998 254 B1**
(45) Veröffentlichungstag und Bekanntmachung des Hinweises auf die Patenterteilung: **24.12.2008**
(21) Anmeldenummer: 98936432.8
(22) Anmeldetag: 20.07.1998
(51) Int. Cl.: A61K 8/894, A61Q 5/06, C08F 283/12

(54) **VERWENDUNG VON POLYSILOXANHALTIGEN POLYMEREN FÜR KOSMETISCHE FORMULIERUNGEN**
USE OF POLYMERS CONTAINING POLYSILOXANE FOR COSMETIC FORMULATIONS
UTILISATION DE POLYMERES CONTENANT DU POLYSILOXANE POUR DES FORMULATIONS COSMETIQUES

(30) Priorität: 23.07.1997 DE 19731529
(43) Veröffentlichungstag der Anmeldung: 10.05.2000
(73) Patentinhaber: BASF SE, 67056 Ludwigshafen (DE)
(72) Erfinder: BLANKENBURG, Rainer, D-67061 Ludwigshafen (DE); DIEING, Reinhold, D-67105 Schifferstadt (DE); MÜLLER, Wolfgang, D-67227 Frankenthal (DE); SANNER, Axel, D-67227 Frankenthal (DE); SCHEHLMANN, Volker, 67105 Schifferstadt (DE); ZEITZ, Katrin, D-67067 Ludwigshafen (DE)
(86) Internationale Anmeldenummer: PCT/EP1998/004482
(87) Internationale Veröffentlichungsnummer: WO 1999/004750

(56) Entgegenhaltungen:
- EP-A- 0 408 311
- EP-A- 0 412 707
- EP-A- 0 582 152
- EP-A- 0 670 342
- WO-A-93/23446
- DE-A- 1 645 569
- FR-A- 2 740 037

## Beschreibung

Für die Festigung von Haarfrisuren werden seit fast 50 Jahren erfolgreich synthetische Polymere eingesetzt. Während in der Anfangszeit bevorzugt Vinyllactam-Homo- und Copolymere zum Einsatz gelangten, haben später carboxylatgruppenhaltige Polymere zunehmend an Bedeutung gewonnen. Das gewünschte Eigenschaftsprofil wie starke Festigung bei hoher Luftfeuchtigkeit, Elastizität, Auswaschbarkeit vom Haar und Verträglichkeit mit den übrigen Formulierungskomponenten werden durch Copolymerisation einer Kombination von hydrophoben, elastifizierenden und carboxylgruppen enthaltenden Monomeren erzielt.

Während die obengenannten Anforderungen heute von verschiedenen Polymertypen erreicht werden, wird immer häufiger der Griff der mit diesen Polymeren gefestigten Frisuren als unangenehm stumpf und "unnatürlich" empfunden. Versuche, durch Zusätze zu den Formulierungen zu einer Verbesserung zu gelangen, führten bisher nicht zu voll befriedigenden Ergebnissen: Die Zugabe üblicher Weichmacher verbessert zwar den Griff, reduziert aber gleichzeitig in vielen Fällen die Festigungswirkung. Die häufig eingesetzten Polysiloxane sind mit den polaren Polymeren nicht verträglich und verlangen oft weitere Zusätze, um überhaupt formuliert werden zu können. Entmischungen können sowohl während der Lagerung der Formulierung als auch während des Gebrauchs zu Problemen führen.

Es hat daher nicht an Versuchen gefehlt, Polysiloxangruppen kovalent an das Festigerpolymer zu binden, um Entmischungen zu verhindern. Beispielsweise beschreibt die europäische Patentanmeldung EP 0408311 Haarpflege-Polymere aus einem Polysiloxangruppen-enthaltendem Monomer und üblichen hydrophilen und hydrophoben Monomeren. In den europäischen Patentanmeldungen EP 0412704 bis EP 0412707 wird vorgeschlagen, Polysiloxangruppen in Form von Makromonomeren mit Molmassen von 1000 bis 50000 mit üblichen hydrophoben und hydrophilen Monomeren zu polymerisieren. Die Synthese dieser Monomeren ist außerordentlich aufwendig. Aus den Polymeren können nicht umgesetzte Makromonomere und deren unreaktive Verunreinigungen aufgrund ihres hohen Molekulargewichts kaum abgetrennt werden Sie stellen ein toxikologisches und allergenes Risiko dar. Darüber hinaus sind die erhaltenen Copolymeren, um eine gute Wirkung zu erzielen, oft nur in Kombination mit weiteren Polymeren, Carriern und weiteren Hilfsmittel zu formulieren, wie die o.g. Patentschriften lehren.

DE 42 40 108 beschreibt polysiloxanhaltige Bindemittel, die sich als schmutzabweisende Überzüge, insbesondere als anti-Graffiti-Überzüge, eignen. Diese Bindemittel sind jedoch lackartig und eignen sich nicht für kosmetische Zwecke.

DE 16 45 569 beschreibt ein Verfahren zur Herstellung von siliconorganischen Pfropfmischpölymeren und deren Verwendung als Schaummassen.

Aufgabe der Erfindung ist die Bereitstellung von Polymeren für die Haarkosmetik ohne die geschilderten Nachteile.

Gefunden wurde die Verwendung von Polymeren, die wasserlöslich oder wasserdispergierbar sind oder die, für den Fall, daß sie aus Monomeren mit neutralisierbaren Resten bestehen, in neutralisierter Form wasserlöslich oder wasserdispergierbar sind, wie in Anspruch 1 definiert.

Mit "wasserdispergierbar" im Sinne der Erfindung sind Polymere gemeint, die im Kontakt mit Wasser innerhalb von 24 Stunden ein Fluid bilden, das ohne optische Hilfsmittel mit dem Auge keine festen Partikel erkennen läßt. Zur Überprüfung, ob ein Polymer wasserdispergierbar ist, werden 100 mg des Polymers in Form eines 100 µm dicken Films in 100 ml Wasser (20°C) gegeben und auf einem handelsüblichen Schütteltisch für 24 Stunden geschüttelt. Wenn nach dem Schütteln keine festen Partikel mehr erkennbar sind, das Fluid aber eine Trübung besitzt, ist das Polymer wasserdispergierbar; ohne Trübung wird es als wasserlöslich bezeichnet.

Sind die Silikonverbindungen nicht während der Polymerisation zugegen, sondern werden nach der Polymerisation eingemischt, so erhält man in der Regel sehr weiche klebrige Filme, die für die erfindungsgemäßen Anwendungen in der Haarkosmetik ungeeignet sind (siehe Vergleichsbeispiele 8 und 24).

Dies deutet darauf hin, daß es während der Polymerisation eventuell zu einer Pfropfung der Polymerisate auf die Silikonverbindungen kommen kann, und dies zu den guten Filmeigenschaften wie Klebfreiheit, hohe Oberflächenglätte und Härte beiträgt. Es sind jedoch auch andere Mechanismen als Pfropfung vorstellbar, durch die die erfindungsgemäßen Polymere zu ihren vorteilhaften Eigenschaften kommen.

Als geeignete polymerisierbare Monomere (a) können bevorzugt ethylenisch ungesättigte Monomere verwendet werden. Dabei kann entweder ein einzelnes Monomer oder Kombinationen von zwei oder mehr Monomeren verwendet werden. Mit polymerisierbar ist gemeint, daß die verwendeten Monomere unter Verwendung irgendeiner konventionellen synthetischen Methode polymerisiert werden können.

Beispielsweise können dies Lösungspolymerisation, Emulsionspolymerisation, umgekehrte Emulsionspolymerisation, Suspensionspolymerisation, umgekehrte Suspensionspolymerisation oder Fällungspolymerisation sein, ohne daß die verwendbaren Methoden darauf beschränkt sind. Bei der Lösungspolymerisation kann Wasser, übliche organische Lösungsmittel oder die erfindungsgemäßen Silikonderivate selbst als Lösungsmittel verwendet werden.

Monomere die mit einer durch freie Radikale initiierten Reaktion polymerisiert werden können sind bevorzugt. Der Begriff ethylenisch ungesättigt bedeutet, daß die Monomere zumindest eine polymerisierbare Kohlenstoff-Kohlenstoff Doppelbindung besitzen, die mono-, di-, tri-, oder tetrasubstituiert sein kann.

Die Monomere (a) der polysiloxanhaltigen Polymere der vorliegenden Erfindung können von 50 bis 99,9 Gew.-% , bevorzugt 70 bis 99 Gew.-%, besonders bevorzugt 85 bis 98 Gew.-% ausmachen.

Die bevorzugten ethylenisch ungesättigten Monomere (a) können durch die folgende allgemeine Formel beschrieben werden:

X-C(O)CR⁷=CHR⁶

wobei
X ausgewählt ist aus der Gruppe der Reste -OH, -OM, -OR⁸, NH₂, -NHR⁸, N(R⁸)₂ ;
M ist ein Kation ausgewählt aus der Gruppe bestehend aus: Na+, K+, Mg++, Ca++, Zn++, NH4+, Alkylammonium, Dialkylammonium, Trialkylammonium und Tetraalkylammonium;
die Reste R⁸ können identisch oder verschieden ausgewählt werden aus der Gruppe bestehend aus -H, C1-C40 linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl.

R⁷ und R⁶ sind unabhängig voneinander ausgewählt aus der Gruppe bestehend aus: -H, C₁-C₈ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren (a) sind zum Beispiel Acrylsäure und deren Salze, Ester und Amide. Die Salze können von jedem beliebigen nicht toxischen Metall, Ammonium oder substituierten Ammonium-gegenionen abgeleitet sein.

Die Ester können abgeleitet sein von C₁-C₄₀ linearen, C₃-C₄₀ verzweigtkettigen, oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin, and 1,2,6-Hexantriol, von Aminoalkoholen oder von Alkoholethern wie Methoxyethanol und Ethoxyethanol oder Polyethylenglykolen.

Ferner eignen sich N,N-Dialkylaminoalkylacrylate- und methacrylate und N-Dialkylaminoalkylacryl- und -methacrylamide der allgemeinen Formel (II) 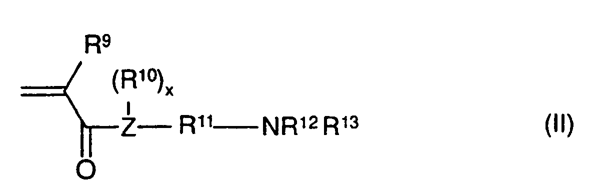
- mit R⁹: = H, Alkyl mit 1 bis 8 C-Atomen,
- R¹⁰: = H, Methyl,
- R¹¹: = Alkylen mit 1 bis 24 C-Atomen, optional substituiert durch Alkyl,
- R¹², R¹³ =: C₁-C₄₀ Alkylrest,
- Z: = Stickstoff für x = 1 oder Sauerstoff für x = 0

Die Amide können unsubstituiert, N-Alkyl oder N-alkylamino monosubstituiert, oder N,N-dialkylsubstituiert oder N,N-dialkylamino disubstituiert, worin die Alkyl- oder Alkylaminogruppen von C1-C40 linearen, C3-C40 verzweigtkettigen, oder C3-C40 carbocyclischen Einheiten abgeleitet sind. Zusätzlich können die Alkylaminogruppen quarternisiert werden.

Bevorzugte Monomere der Formel II sind N,N-Dimethylaminomethyl-(meth)acrylat, N,N-Diethylaminomethyl(meth)acrylat, N,N-Dimethylaminoethyl(meth)acrylat, N,N-Diethylaminoethyl(meth)acrylat.

Ebenfalls verwendbare Monomere (a) sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe bestehend aus C1-C4 Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Monomere (a) sind Vinyl- und Allylester von C1-C40 linearen, C3-C40 verzweigtkettigen oder C3 - C40 carbocyclische Carbonsäuren (z.B.: Vinylacetat, Vinylpropionat, Vinylneononanoat, Vinylneoundekansäure oder t-Butyl-benzoesäure-vinylester); Vinyl- oder Allylhalogenide, bevorzugt Vinylchlorid und Allylchlorid, Vinylether, bevorzugt Methyl-, Ethyl-, Butyl-, oder Dodecylvinylether, Vinylformamid, Vinylmethylacetamid, Vinylamin; Vinyllactame, bevorzugt Vinylpyrrolidon und Vinylcaprolactam, Vinyl- oder Allyl-substituierte heterocyclische Verbindungen, bevorzugt Vinylpyridin, Vinyloxazolin und Allylpyridin.

Weiterhin sind N-Vinylimidazole der allgemeinen Formel III geeignet, worin R¹⁴ bis R¹⁶ unabhängig voneinander für Wasserstoff, C₁-C₄-Alkyl oder Phenyl steht: 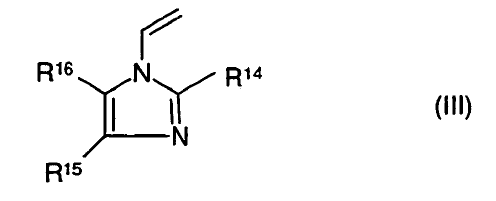 Weitere geeignete Monomere (a) sind Diallylamine der allgemeinen Formel (IV) 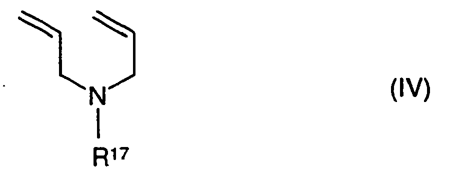 mit R¹⁷= C1 bis C24 Alkyl

Weitere geeignete Monomere (a) sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere.

Besonders geeignete Monomere (a) sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butyl-ethacrylat, 2-Ethylhexylethacrylat, Decylethacrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren wie zum Beispiel Acrylamidopropansulfonsäure;

Acrylamid, Methacrylamid, Ethacrylamid, N-Methylacrylamid, N,N-Dimethylacrylamid, N-Ethylacrylamid, N-Isopropylacrylamid, N-Butylacrylamid, N-t-Butylacrylamid, N-Octylacrylamid, N-t-Octylacrylamid, N-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylimidazol, N,N-Dimethylaminomethyl(meth)acrylat, N,N-Diethylaminomethyl(meth)acrylat, N,N-Dimethylaminoethyl(meth)acrylat, N,N-Diethylaminoethyl(meth)-acrylat, N,N-Dimethylaminobutyl(meth)acrylat, N,N-Diethylaminobutyl(meth)acrylat, N,N-Dimethylaminohexyl(meth)acrylat, N,N-Dimethylaminooctyl(meth)acrylat, N,N-Dimethylaminododecyl(meth)-acrylat, N-[3-(dimethylamino)propyl]methacrylamid, N-[3-(dimethylamino)propyl]acrylamid, N-[3-(dimethylamino)butyl]methacrylamid, N-[8-(dimethylamino)octyl]methacrylamid, N-[12-(dimethylamino)dodecyl]methacrylamid, N-[3-(diethylamino)propyl]-methacrylamid, N-[3-(diethylamino)propyl]acrylamid;

Maleinsäure, Fumarsäure, Maleinsäureanhydrid und seine Halbester, Crotonsäure, Itaconsäure, Diallyldimethylammoniumchlorid, Vinylether (zum Beispiel: Methyl-, Ethyl-, Butyl-, oder Dodecylvinylether), Vinylformamid, Vinylmethylacetamid, Vinylamin; Methylvinylketon, Maleimid, Vinylpyridin, Vinylimidazol, Vinylfuran, Styrol, Styrolsulfonat, Allylalkohol, und Mischungen daraus.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, N-t-Butylacrylamid, N-Octylacrylamid, 2-Hydroxyethylacrylat, Hydroxypropylacrylat, 2-Hydroxyethylmethacrylat, Hydroxypropylmethacrylat, Alkylenglykol(meth)-acrylate, ungesättigte Sulfonsäuren wie zum Beispiel Acrylamidopropansulfonsäure, Vinylpyrrolidon, Vinylcaprolactam, Vinylether (z.B.: Methyl-, Ethyl-, Butyl-, oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N-[3-(dimethylamino)propyl]methacrylamid ; 3-Methyl-1-vinylimidazoliumchlorid, 3-Methyl-1-vinylimidazoliummethylsulfat, N,N-Dimethylaminoethylmethacrylat, N-[3-(dimethylamino)propyl]-methacrylamid quaternisiert mit Methylchlorid, Methylsulfat oder Diethylsulfat.

Monomere, mit einem basischen Stickstoffatom, können dabei auf folgende Weise quarternisiert werden:

Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der basischen Amine kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.

Die Quaternisierung kann vor der Polymerisation oder nach der Polymerisation durchgeführt werden.

Außerdem können die Umsetzungsprodukte von ungesättigten Säuren, wie z.B. Acrylsäure oder Methacrylsäure, mit einem quaternisierten Epichlorhydrin der allgemeinen Formel (V) eingesetzt werden (R¹⁸ = C 1 bis C 40 Alkyl).

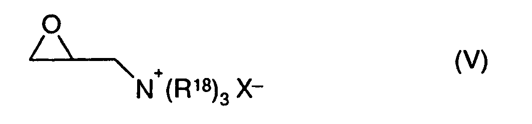

Beispiele hierfür sind zum Beispiel: (Meth)acryloyloxyhydroxypropyltrimethylammoniumchlorid und (Meth)acryloyloxyhydroxypropyltriethylammoniumchlorid

Die basischen Monomere können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Zusätzlich zu den oben genannten Monomeren können als Monomere (a) sogenannte Makromonomere wie zum Beispiel silikonhaltige Makromonomere mit ein oder mehreren radikalisch polymerisierbaren Gruppen oder Alkyloxazolinmakromonomere eingesetzt werden wie sie zum Beispiel in der EP 408 311 beschrieben sind.

Des weiteren können fluorhaltige Monomere wie sie beispielsweise in der EP 558423 beschrieben sind, vernetzend wirkende oder das Molekulargewicht regelnde Verbindungen in Kombination oder alleine eingesetzt werden.

Als Regler können die üblichen dem Fachmann bekannten Verbindungen wie zum Beispiel Schwefelverbindungen (z.B.: Mercaptoethanol, 2-Ethylhexylthioglykolat, Thioglykolsäure oder Dodecylmercaptan) sowie Tribromchlormethan oder andere Verbindungen die regelnd auf das Molekulargewicht der erhaltenen Polymerisate wirken, verwendet werden.

Es können gegebenenfalls auch thiolgruppenhaltige Silikonverbindungen eingesetzt werden.
Bevorzugt werden silikonfreie Regler eingesetzt.

Als vernetzende Monomere können Verbindungen mit mindestens zwei ethylenisch ungesättigten Doppelbindungen eingesetzt werden wie zum Beispiel Ester von ethylenisch ungesättigten Carbonsäuren, wie Acrylsäure oder Methacrylsäure und mehrwertigen Alkoholen, Ether von mindestens zweiwertigen Alkoholen wie zum Beispiel Vinylether oder Allylether. Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen. Ferner geeignet sind Amide der Acryl- und Methacrylsäure und N-Allylamine von mindestens zweiwertigen Aminen wie zum Beispiel (1,2-Diaminoethan, 1,3-Diaminopropan). Ferner sind Triallylamin oder entsprechende Ammoniumsalze, N-Vinylverbindungen von Harnstoffderivaten, mindestens zweiwertigen Amiden, Cyanuraten oder Urethanen. Weitere geeignete Vernetzer sind Divinyldioxan, Tetraallylsilan oder Tetravinylsilan.

Besonders bevorzugte Vernetzer sind beispielsweise Methylenbisacrylamid, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen die mit Ethylenoxid und/oder Propylenoxid und/oder Epichlorhydrin umgesetzt worden sind.

Bei der Polymerisation der Monomeren (a) können gegebenenfalls auch andere Polymere wie zum Beispiel Polyamide, Polyurethane, Polyester, Homo- und Copolymere von ethylenisch ungesättigten Monomeren zugegen sein. Beispiele für solche zum Teil auch in der Kosmetik eingesetzten Polymeren sind die unter den Handelsnamen bekannten Polymere Amerhold^{™}, Ultrahold ^{™}, Ultrahold Streng^{™}, Luviflex^{™} VBM, Luvimer^{™}, Acronal^{™}, Acudyne^{™}, Stepanhold^{™}, Lovocryl^{™}, Versatyl^{™}, Amphomer^{™} oder Eastma AQ^{™}.

Solche oder andere Polymere können auch nach der Polymerisation den erfindungsgemäßen Polymerzubereitungen beigemischt werden.

Die erfindungsgemäßen Monomere A können, sofern sie ionisierbare Gruppen enthalten, vor oder nach der Polymerisation, zum Teil oder vollständig mit Säuren oder Basen neutralisiert werden um so zum Beispiel die Wasserlöslichkeit oder -dispergierbarkeit auf ein gewünschtes Maß einzustellen.
Als Neutralisationsmittel für Säuregruppen tragende Monomere können zum Beispiel Mineralbasen wie Natriumcarbonat, Alkalihydroxide sowie Ammoniak, organische Basen wie Aminoalkohole speziell 2-Amino-2-Methyl-1-Propanol, Monoethanolamin, Diethanolamin, Triethanolamin, Triisopropanolamin, Tri[(2-hydroxy)1-Propyl] amin, 2-Amino-2-Methyl-1,3-Propandiol, 2-Amino-2-hydroxymethyl-1,3-Propandiol sowie Diamine wie zum Beispiel Lysin verwendet werden.
Als Neutralisationsmittel für kationisierbare Gruppen tragende Monomere können zum Beispiel Mineralsäuren wie Salzsäure, Schwefelsäure oder Phosphorsäure, sowie organische Säuren wie Carbonsäuren Milchsäure, Zitronensäure oder andere eingesetzt werden.

Es können weiterhin Hilfsstoffe wie Weichmacher, Filmbildehilfsmittel, Pigmente, Parfums oder andere, alleine oder in Kombination, bei der Polymerisation anwesend sein und/oder nach der Polymerisation zugefügt werden.

Bei Verwendung der erfindungsgemäßen Polymere in der Haarkosmetik, speziell bei der Verwendung als Festiger, ist es vorteilhaft die Glastemperatur der Polymerisate durch geeignete Kombination von ethylenisch ungesättigten Monomeren auf Werte größer 20 °C einzustellen.

Geeignete Silikonderivate (b) sind die unter dem INCI Namen Dimethicone Copolyole oder Silikontenside bekannten Verbindungen wie zum Beispiel die unter den Markennamen Abil^{®} (der Fa. T. Goldschmidt), Alkasil^{®} (der Fa. Rhöne-Poulenc), Silicone Polyol Copolymer^{®} (der Fa Genesee), Belsil^{®} (der Fa. Wacker), Silwet^{®} (der Fa. Witco, Greenwich, CT, USA) oder Dow Corning (der Fa. Dow Corning) erhältlich Diese beinhalten Verbindungen mit den CAS-Nummern 64365-23-7; 68937-54-2; 68938-54-5; 68937-55-3.

Monomere (b) sind solche, die die folgenden Strukturelemente enthalten: 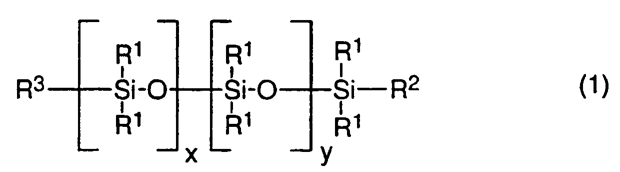 wobei:
R² = CH₃ oder 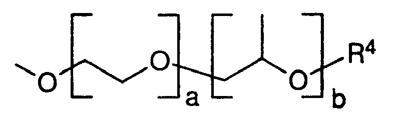 R³ = CH₃ oder R²
R⁴ = H, CH₃, 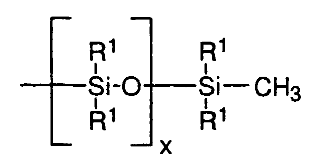 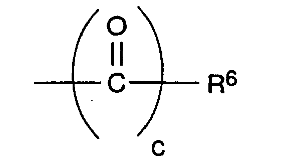
- R⁶: ein organischer Rest aus 1 bis 40 Kohlenstoffatomen, der Amino-, Carbonsäure- oder Sulfonatgruppen enthalten kann oder für den Fall c=O, auch das Anion einer anorganischen Säure bedeutet,
und wobei die Reste R¹ identisch oder unterschiedlich sein können, und werden aus der folgenden Gruppe ausgewählt: Methyl, Ethyl,Propyl, Butyl, Isobutyl, Pentyl, Isopentyl, Hexyl, Octyl, Decyl, Dodecyl und Octadecyl, Cyclohexyl, aromatische Gruppen, speziell Phenyl oder Naphthyl, Benzyl, Phenylethyl sowie Tolyl und Xylyl oder gleich R₅ sind, wobei: 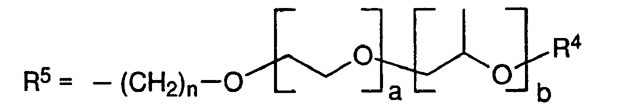 mit der Maßgabe, daß mindestens einer der Reste R¹, R² oder R³ ein polyalkylenoxidhaltiger Rest nach obengenannter Definition ist, und n eine ganze Zahl von 1 bis 6 ist,
x und y ganze Zahlen derart sind, daß das Molekulargewicht des Polysiloxan-Blocks zwischen 300 und 30000 liegt,
a,b ganze Zahlen zwischen 0 und 50 sein können mit der Maßgabe, daß die Summe aus a und b größer als 0 ist, und C 0 oder 1 ist.

Bevorzugte Reste R² und R⁵ sind solche, bei denen die Summe aus a+b zwischen 5 und 30 beträgt.

Besonders geeignete Reste R⁴ sind solche, bei denen im Falle von R⁴ = -(CO)_{c}-R⁶ R⁶ ein beliebiger Alkyl-, Cycloalkyl oder Arylrest bedeutet, der zwischen 1 und 40 C-Atomen besitzt und der weitere ionogene Gruppen wie NH₂, COOH, SO₃H tragen kann.

Bevorzugte anorganische Reste R⁶ sind, für den Fall c=O, Phosphat und Sulfat.

Besonders bevorzugte Silikonderivate (b) sind solche, der allgemeinen Struktur:

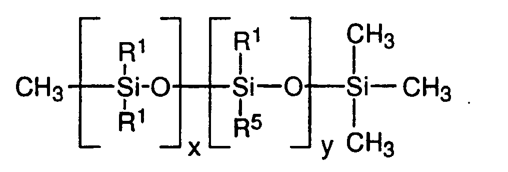

Die Silikonderivate (b) sind in der Regel in Mengen von 0,1-50, bevorzugt von 1-20, besonders bevorzugt von 2-15 Gew.-% in dem erfindungsgemäßen Polymerisat enthalten.

Besonders geeignete Polymere sind solche, die wasserlöslich sind oder deren Wasserdispergierbarkeit so groß ist, daß sie in einem Lösungsmittelgemisch Wasser:Ethanol = 50:50 (Vol.-%:Vol.-%) in einer Menge von mehr als 0,1 g/1, bevorzugt mehr als 0,2 g/L, löslich sind.

Für den Fall, daß die Polymere aus Monomeren bestehen, die neutralisierbare Reste tragen, sind solche Polymere bevorzugt, die in der neutralisierten Form in einem Lösungsmittelgemisch Wasser:Ethanol = 50:50 (Vol.-%:Vol.-%) in einer Menge von mehr als 0,1 g/1, bevorzugt mehr als 0,2 g/L, löslich sind.

Die erfindungsgemäßen Polymere eignen sich zur Verwendung als Wirkstoffe in kosmetischen Zubereitungen, seien es hautkosmetische Zubereitungen wie Flüssigseifen, Körperlotionen, Rasierwasser, Gesichtswasser, Deodorantien und andere kosmetische Lotionen oder, vor allem haarkosmetische Zubereitungen wie Haarkuren, Haarlotionen, Haarspülungen, Haaremulsionen, Spitzenfluids, Egalisierungsmittel für Dauerwellen, Hot-Oil-Treatment-Präparate, Conditioner, Festigerlotionen oder Haarsprays. Je nach Anwendungsgebiet können die haarkosmetischen Zubereitungen als Spray, Schaum, Gel, Gelspray, Lotion oder Mousse appliziert werden.

Die erfindungsgemäßen Mischungen können mit den für Kosmetikpräparaten üblichen Hilfsmitteln wie Parfümöle, Emulgatoren, Konservierungsmittel, Pflegestoffe wie Panthenol, Collagen, Vitamine, Eiweißhydrolysate, Stabilisatoren, pH-Wert Regulatoren, Farbstoffe, Lösemitteln, Treibgasen und weiteren üblichen Additiven zu Gelen, Sprays, Lotionen oder Schäumen verarbeitet werden.

### Beispiele

Die in den Beispielen verwendeten Silikontenside Wacker Belsil^{™} DMC 6031 und 6032 sind erhältlich bei der Fa. Wacker Chemie GmbH, München und haben die folgende allgemeine Struktur: 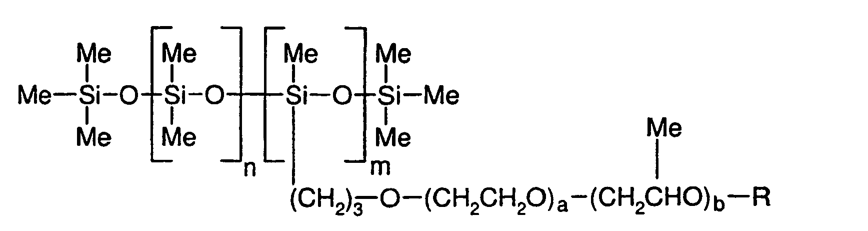 mit R = H, -CO-CH₃

Die Silikontenside Silwet^{™} 7600, 7604 und 7605 sind erhältlich von der Firma Witco Corporation, Greenwich, CT, USA und weisen die folgende allgemeine Struktur auf: 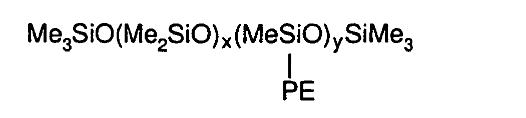 PE =-CH₂CH₂CH₂O(EO)ₘ(PO)ₙZ
Z = Wasserstoff- oder Alkylradikal

Die Silikontenside Dow Corning 190 Surfactant^{™} sind erhältlich von der Firma Dow Corning Corporation, Midland, MI, USA.

Weitere Silikonderivate (b) können nach dem Fachmann geläufigen Verfahren hergestellt werden, wie sie beispielsweise in EP 775 717 beschrieben sind.

### Beispiele 1-7

Zu einer gerührten Vorlage werden 50 g Zulauf 1 und 3,75 g Zulauf 2 getropft. Die Mischung wird dann auf 78°C erhitzt. Danach werden innerhalb 1,5 h der Rest von Zulauf 1 und von Zulauf 2 zugetropft. Die Mischung wird weitere 2 h gerührt. Danach wird Zulauf 3 innerhalb von 15 min. zugetropft und noch 3 h bei 78°C gerührt.

### Beispiel 1

| | |
|---|---|
| Vorlage: | 175 g Ethanol, 7,5 g Dow Corning 190^{™} |
| Zulauf 1: | 251 g t-Butylacrylat, 86 g Methacrylsäure, 37 g Ethylacrylat, 75 g Ethanol |
| Zulauf 2: | 2 g t-Butyl-perpivalat, 100 g Ethanol |
| Zulauf 3: | 1,5 g t-Butyl-perpivalat, 57 g Ethanol |

### Beispiel 2

| | |
|---|---|
| Vorlage: | 175 g Ethanol, 18,75 g Dow Corning 190^{™} |
| Zulauf 1: | 251 g t-Butylacrylat, 86 g Methacrylsäure, 37 g Ethylacrylat, 75 g Ethanol |
| Zulauf 2: | 2 g t-Butyl-perpivalat, 100 g Ethanol |
| Zulauf 3: | 1,5 g t-Butyl-perpivalat, 57 g Ethanol |

### Beispiel 3

| | |
|---|---|
| Vorlage: | 175 g Ethanol, 37,5 g Dow Corning 190^{™} |
| Zulauf 1: | 251 g t-Butylacrylat, 86 g Methacrylsäure, 37 g Ethylacrylat, 75 g Ethanol |
| Zulauf 2: | 2 g t-Butyl-perpivalat, 100 g Ethanol |
| Zulauf 3: | 1,5 g t-Butyl-perpivalat, 57 g Ethanol |

### Beispiel 4

| | |
|---|---|
| Vorlage: | 175 g Ethanol, 18,75 g Belsil DMC 6031^{™} |
| Zulauf 1: | 251 g t-Butylacrylat, 86 g Methacrylsäure, 37 g Ethylacrylat, 75 g Ethanol |
| Zulauf 2: | 2 g t-Butyl-perpivalat, 100 g Ethanol |
| Zulauf 3: | 1,5 g t-Butyl-perpivalat, 57 g Ethanol |

### Beispiel 5

| | |
|---|---|
| Vorlage: | 175 g Ethanol, 37,5 g Belsil DMC 6031^{™} |
| Zulauf 1: | 279 g t-Butylacrylat, 96 g Methacrylsäure, 75 g Ethanol |
| Zulauf 2: | 2 g t-Butyl-perpivalat, 100 g Ethanol |
| Zulauf 3: | 1,5 g t-Butyl-perpivalat, 57 g Ethanol |

### Beispiel 6

| | |
|---|---|
| Vorlage: | 175 g Ethanol, 37,5 g Belsil DMC 6032^{™} |
| Zulauf 1: | 300 g t-Butylacrylat, 75 g Methacrylsäure, 75 g Ethanol |
| Zulauf 2: | 2 g t-Butyl-perpivalat, 100 g Ethanol |
| Zulauf 3: | 1,5 g t-Butyl-perpivalat, 57 g Ethanol |

### Beispiel 7 (Vergleichsbeispiel)

| | |
|---|---|
| Vorlage: | 175 g Ethanol |
| Zulauf 1: | 251 g t-Butylacrylat, 86 g Methacrylsäure, 37 g Ethylacrylat, 75 g Ethanol |
| Zulauf 2: | 2 g t-Butyl-perpivalat, 100 g Ethanol |
| Zulauf 3: | 1,5 g t-Butyl-perpivalat, 57 g Ethanol |

### Beispiel 8 (Vergleichsbeispiel)

Nach der Polymerisation wird die Lösung des Polymeren aus Beispiel 7 mit 10 Gew. % bezogen auf das enthaltene Polymer des Silikonderivates Belsil DMC 6032^{™} abgemischt.

### Beispiel 9

In einer Rührapparatur wurden 40 g Silwet^{™} L 7604 und 180 g Wasser vorgelegt. Unter Rühren im Stickstoffstrom wurde auf 60°C aufgeheizt und Zulauf 1, bestehend aus 240 g N-Vinylpyrrolidon, 267 g 3-Methyl-1-vinylimidazoliummethylsulfat-Lösung (45 prozentig) und 0,4 g Mercaptoethanol, und Zulauf 2, bestehend aus 6 g 2,2'-Azobis(2-amidinopropan)dihydrochlorid und 70 ml Wasser, innerhalb von 6 Stunden zudosiert. Anschließend wurde noch 2 Stunden bei 60°C gerührt und mit 200 g Wasser verdünnt. Man erhielt eine klare, gelbliche Polymerlösung mit einem Feststoffgehalt von 41,7 % und einem K- Wert von 39 (1 %ig in 0,5 m NaCl).

### Beispiel 10

In einer Rührapparatur wurden 40 g Silwet^{™} L 7604und 300 ml Wasser vorgelegt. Unter Rühren im Stickstoffstrom wurde auf 65°C aufgeheizt und Zulauf 1, bestehend aus 220 g N-Vinylpyrrolidon und 333 g 3-Methyl-1-vinylimidazoliumchlorid-Lösung (60 prozentig) und Zulauf 2, bestehend aus 6 g 2,2'-Azobis(2-amidinopropan)dihydrochlorid und 70 ml Wasser, innerhalb von 6 Stunden zudosiert. Anschließend wurde noch 2 Stunden gerührt und mit 100 g Wasser verdünnt. Man erhielt eine klare, gelbliche Polymerlösung mit einem Feststoffgehalt von 43,0 % und einem K-Wert von 44 (1 %ig in 0,5 m NaCl) .

### Beispiel 11

In einer Rührapparatur wurden 24 g Silwet^{™} L 7604 und 200 ml Wasser vorgelegt. Unter Rühren im Stickstoffstrom wurde auf 65°C aufgeheizt und Zulauf 1, bestehend aus 160 g N-Vinylpyrrolidon, 80 g Methacryloxyethyl-N-dimethyl-N-ethylammonium-ethylsulfat und 300 g Wasser, und Zulauf 2, bestehend aus 1,3 g 2,2'-Azobis-(2-amidinopropan)dihydrochlorid und 100 g Wasser, innerhalb von 6 Stunden zudosiert. Anschließend wurde noch 2 Stunden gerührt und mit 300 g Wasser verdünnt. Man erhielt eine klare, gelbliche Polymerlösung mit einem Feststoffgehalt von 22,4 % und einem K-Wert von 85 (1 %ig in 0,5 m NaCl).

### Beispiele 12 und 13

In einer Mischung aus 1,4 g Mercaptoethanol, 5,8 g einer Polyacrylsäure (erhältlich unter der Bezeichnung Sokalan PA 110 S von der Firma BASF-AG) werden 306 g N-t-Butyl-acrylamid, 234 g Ethylacrylat, 60 g Acrylsäure und 60 g eines Dimethiconcopolyols suspendiert. Zu der so erhaltenen Suspension werden nach Aufheizen auf 75°C 1,2 g t-Butyl-peroctoat gegeben. Nach 30 bzw. 45 min wird jeweils ein weiteres Gramm t-Butyl-peroctoat zugegeben. Nach folgenden Zeiten wird weiterer Initiator zugegeben: 1 h: 1 g bei 80°C; 1,5 h: 1 g; 2 h: 1 g bei 90°C; 3 h: 2,7 g; nach 4,5 und 6 h je 1 g. Dann wird noch 1 h nachpolymerisiert.

Als Dimethiconpolyole wurden verwendet:
Beispiel 12: Wacker Belsil DMC 6031
Beispiel 13a:Wacker Belsil DMC 6032
Beispiel 13b:Witco Silwet^{®} L-7500

### Beispiel 14 (Vergleichsbeispiel)

In einer Mischung aus 1,4 g Mercaptoethanol, 5,8 g einer Polyacrylsäure (erhältlich unter der Bezeichnung Sokalan PA 110 S von der Firma BASF-AG) werden 306 g N-t-Butyl-acrylamid, 234 g Ethylacrylat und 60 g Acrylsäure suspendiert. Zu der so erhaltenen Suspension werden nach Aufheizen auf 75°C 1,2 g t-Butyl-peroctoat gegeben. Nach 30 bzw. 45 min wird jeweils ein weiteres Gramm t-Butyl-peroctoat zugegeben. Nach folgenden Zeiten wird weiterer Initiator zugegeben: 1 h: 1 g bei 80°C; 1,5 h: 1 g; 2 h: 1 g bei 90°C; 3h: 2,7 g; nach 4,5 und 6h je 1 g. Dann wird noch 1 h nachpolymerisiert.

### Beispiel 15

Ein Gemisch aus 744 g Wasser, 0,25 g Natrium-Laurylsulfat und 70 g Zulauf 1 wird auf 40°C aufgeheizt. Dann werden 16 g einer 7 %igen wäßrigen Natrium-Persulfat Lösung zugegeben. Danach wird die Mischung auf 80°C aufgeheizt und Zulauf 1 während zwei Stunden zudosiert. Dann werden 178 g Wasser zugegeben, und es werden zwei Stunden bei 80°C nachpolymerisiert.

| | |
|---|---|
| Zulauf 1: | 300 g Wasser |
| | 2 g Natrium-Laurylsulfat |
| | 15,7 g Silikoncopolyol (Silwet L7605) |
| | 470 g tert. Butylacrylat |
| | 70 g Ethylacrylat |
| | 161 g Methacrylsäure |
| | 3 g Ethyl-hexylthioglykolat |

### Beispiel 16

Ein Gemisch aus 744 g Wasser, 0,25 g Natrium-Laurylsulfat und 70 g Zulauf 1 wird auf 40°C aufgeheizt. Dann werden 16 g einer 7 % igen wäßrigen Natrium-Persulfat Lösung zugegeben. Danach wird die Mischung auf 80°C aufgeheizt und Zulauf 1 während zwei Stunden zudosiert. Dann werden 178 g Wasser zugegeben, und es werden zwei Stunden bei 80°C nachpolymerisiert.

| | |
|---|---|
| Zulauf 1: | 300 g Wasser |
| | 2 g Natrium-Laurylsulfat |
| | 15,7 g PEO(20)-Sorbitanmonooleat |
| | 422 g tert. Butylacrylat |
| | 63 g Ethylacrylat |
| | 145 g Methacrylsäure |
| | 3 g Ethyl-hexylthioglykolat |
| | 70 g Silikoncopolyol (Silwet^{™} L7605) |

### Beispiel 17

Ein Gemisch aus 744 g Wasser, 0,25 g Natrium-Laurylsulfat und 70 g Zulauf 1 wird auf 40°C aufgeheizt. Dann werden 16 g einer 7 % igen wäßrigen Natrium-Persulfat Lösung zugegeben. Danach wird die Mischung auf 80°C aufgeheizt und Zulauf 1 während zwei Stunden zudosiert. Dann werden 178 g Wasser zugegeben, und es werden zwei Stunden bei 80°C nachpolymerisiert.

| | |
|---|---|
| Zulauf 1: | 300 g Wasser |
| | 2 g Natrium-Laurylsulfat |
| | 15,7 g Silikoncopolyol (Silwet^{™} L7600) |
| | 470 g tert. Butylacrylat |
| | 70 g Ethylacrylat |
| | 161 g Methacrylsäure |
| | 3 g Ethyl-hexylthioglykolat |

### Beispiel 18

Ein Gemisch aus 744 g Wasser, 0,25 g Natrium-Laurylsulfat und 70 g Zulauf 1 wird auf 40°C aufgeheizt. Dann werden 16 g einer 7 % igen wäßrigen Natrium-Persulfat Lösung zugegeben. Danach wird die Mischung auf 80°C aufgeheizt und Zulauf 1 während zwei Stunden zudosiert. Dann werden 178 g Wasser zugegeben, und es werden zwei Stunden bei 80°C nachpolymerisiert.

| | |
|---|---|
| Zulauf 1: | 300 g Wasser |
| | 2 g Natrium-Laurylsulfat |
| | 15,7 g PEO(20)-Sorbitanmonooleat |
| | 422 g tert. Butylacrylat |
| | 63 g Ethylacrylat |
| | 145 g Methacrylsäure |
| | 3 g Ethyl-hexylthioglykolat |
| | 70 g Silikoncopolyol (Silwet^{™} L7605) |

### Beispiel 19

Ein Gemisch aus 744 g Wasser, 0,25 g Natrium-Laurylsulfat und 70 g Zulauf 1 wird auf 40°C aufgeheizt. Dann werden 16 g einer 7 % igen wäßrigen Natrium-Persulfat Lösung zugegeben. Danach wird die Mischung auf 80°C aufgeheizt und Zulauf 1 während zwei Stunden zudosiert. Dann werden 178 g Wasser zugegeben, und es werden zwei Stunden bei 80°C nachpolymerisiert.

| | |
|---|---|
| Zulauf 1: | 300 g Wasser |
| | 2 g Natrium-Laurylsulfat |
| | 15,7 g PEO(20)-Sorbitanmonooleat (Tween 80) |
| | 422 g tert. Butylacrylat |
| | 63 g Ethylacrylat |
| | 145 g Methacrylsäure |
| | 3 g Ethyl-hexylthioglykolat |
| | 70 g Silikoncopolyol (Silwet^{™} L7600) |

### Beispiel 20 (Vergleichsbeispiel)

Ein Gemisch aus 744 g Wasser, 0,25 g Natrium-Laurylsulfat und 70 g Zulauf 1 wird auf 40°C aufgeheizt. Dann werden 16 g einer 7 % igen wäßrigen Natrium-Persulfat Lösung zugegeben. Danach wird die Mischung auf 80°C aufgeheizt und Zulauf 1 während zwei Stunden zudosiert. Dann werden 178 g Wasser zugegeben, und es werden zwei Stunden bei 80°C nachpolymerisiert.

| | |
|---|---|
| Zulauf 1: | 300 g Wasser |
| | 2 g Natrium-Laurylsulfat |
| | 15,7 g PEO(20)-Sorbitanmonooleat (Tween 80) |
| | 470 g tert. Butylacrylat |
| | 70 g Ethylacrylat |
| | 161 g Methacrylsäure |
| | 3 g Ethyl-hexylthioglykolat |

### Beispiel 21

Eine Mischung aus 100 g Vinylcaprolactam, 100 g Ethanol, 100 g Dimethiconcopolyol (Wacker Belsil^{™} DMC 6031) und 0,75 g t-Butylperpivalat werden in einem mit Stickstoff gespülten geschlossenen Kessel auf 70°C aufgeheizt. Dann wird Zulauf 1 innerhalb von 3 h und Zulauf 2 innerhalb 4 h zudosiert. Nach dem Ende von Zulauf 1 wird 1 h nachpolymerisiert. Dann wird Zulauf 3 zugegeben und auf 130°C unter Druck aufgeheizt. Es wird 10 h bei 130°C nachpolymerisiert und abgekühlt.

| | |
|---|---|
| Zulauf 1: | 800 g Vinylcaprolactam |
| | 347 g Ethanol |
| Zulauf 2: | 1,5 g t-Butylperpivalat |
| | 100 g Ethanol |
| Zulauf 3: | 5g Di-t-Butylperoxid |
| | 187 g Ethanol |

### Beispiel 22

Eine Mischung aus 100 g Vinylcaprolactam, 100 g Ethanol, 100 g Dimethiconcopolyol (Wacker Belsil^{™} DMC 6032) und 0,75 g t-Butylperpivalat werden in einem mit Stickstoff gespülten geschlossenen Kessel auf 70°C aufgeheizt. Dann wird Zulauf 1 innerhalb von 3 h und Zulauf 2 innerhalb 4 h zudosiert. Nach dem Ende von Zulauf 1 wird 1 h nachpolymerisiert Dann wird Zulauf 3 zugegeben und auf 130°C unter Druck aufgeheizt. Es wird 10h bei 130°C nachpolymerisiert und abgekühlt.

| | |
|---|---|
| Zulauf 1: | 800 g Vinylcaprolactam |
| | 347 g Ethanol |
| Zulauf 2: | 1,5 g t-Butylperpivalat |
| | 100 g Ethanol |
| Zulauf 3: | 5g Di-t-Butylperoxid |
| | 187 g Ethanol |

### Beispiel 23 (Vergleichsbeispiel)

Eine Mischung aus 100 g Vinylcaprolactam, 100 g Ethanol und 0,75 g t-Butylperpivalat werden in einem mit Stickstoff gespülten, geschlossenen Kessel auf 70°C aufgeheizt. Dann wird Zulauf 1 innerhalb von 3 h und Zulauf 2 innerhalb 4 h zudosiert. Nach dem Ende von Zulauf 1 wird 1 h nachpolymerisiert. Dann wird Zulauf 3 zugegeben und auf 130°C unter Druck aufgeheizt. Es wird 10 h bei 130°C nachpolymerisiert und abgekühlt.

| | |
|---|---|
| Zulauf 1: | 800 g Vinylcaprolactam |
| | 347 g Ethanol |
| Zulauf 2: | 1,5 g t-Butylperpivalat |
| | 100 g Ethanol |
| Zulauf 3: | 5g Di-t-Butylperoxid |
| | 187 g Ethanol |

### Beispiel 24

Die Polymerlösung aus Beispiel 23 wurde nach der Polymerisation mit 10 Ges.-% eines Silikontensids (Wacker Belsil^{™} DMC 6032) abgemischt.

Filme der Polymere aus den Beispielen wurden durch Aufrakeln der Lösungen oder Dispersionen der Polymere auf Glasplatten hergestellt. An diesen Filmen wurde die Transparenz sowie die Oberflächenrauhigkeit und das Reibungsverhalten der Polymerfilme bestimmt (siehe Tabelle 1) .

Dabei zeigt sich, daß alle erfindungsgemäßen Filme transparent sind, und eine besonders glatte Oberfläche mit geringem Reibungswiderstand aufweisen.

Filme aus dem Vergleichsbeispiel 7 sind ebenfalls transparent, zeigen jedoch eine rauhere Oberfläche und vor allem auch einen sehr viel höheren Reibungswiderstand. Das gleiche schlechte Reibungsverhalten wird bei Filmen aus den Beispielen 8, 14, 20, 23 und 24 gefunden. Aus den Abmischungen der Beispiele 8 und 24 in analoger Weise hergestellte Filme zeigen eine extrem hohe Klebrigkeit. Die Filme sind sehr weich und zur Verwendung als Filmbildner daher ungeeignet.

Prüfungen der Polymere 1-24 auf Ihre Eignung als Haarbehandlungsmittel zeigen entsprechende Ergebnisse (Tabelle 1). Dazu werden Haartressen mit einer definierten Menge aus einer Standardformulierung (2 Gew.% des Polymeren, 40 % Dimethylether, 58% Ethanol) der Polymere eingesprüht. Nach dem Trocknen der Haartressen wird die Kämmbarkeit und der Griff an diesen Haarsträhnen beurteilt. Die in Gegenwart von Silikontensiden hergestellten erfindungsgemäßen Polymerisate ergeben einen deutlich besseren Griff der behandelten Haarsträhnen als mit den Vergleichspolymeren 7, 14, 20, 23 behandelte Haarsträhnen.

**Tabelle 1**

| Beispiel | Glätte des Films | Transparenz | Kämmbarkeit | Griff |
|---|---|---|---|---|
| 1 | ○ | + | ○ | ○ |
| 2 | + | + | + | - |
| 3 | + | + | + | ○ |
| 4 | + | + | + | ○ |
| 5 | ++ | + | ++ | ++ |
| 6 | + | + | ++ | ○ |
| 7 | - - | + | - | - - |
| 8 | - - | ○ | -* | --* |
| 12 | + | + | ++ | ++ |
| 13a | + | + | ++ | + |
| 13b | + | + | ++ | + |
| 14 | - | + | ++ | ○ |
| 21 | + | + | + | + |
| 22 | + | + | ++ | + |
| 23 | - | + | ○ | - |
| 24 | - - | - | -* | -* |

| | | | | |
|---|---|---|---|---|
| * aufgrund der viel zu hohen Klebrigkeit wurde auf einen Test an Haartressen verzichtet | | | | |

Die Einträge in der Tabelle haben dabei die folgende Bedeutung:
++ = sehr gute Performance
+ = gute Performance
○ = zufriedenstellende Performance
- = nicht zufriedenstellend
-- = unzureichend

## Patentansprüche

1. Verwendung von Polymeren, die wasserlöslich oder wasserdispergierbar sind oder die, für den Fall, daß sie aus Monomeren mit neutralisierbaren Resten bestehen, in neutralisierter Form wasserlöslich oder wasserdispergierbar sind, die erhältlich sind indem man
(a) ethylenisch ungesättigte Monomere in Gegenwart von
(b) polyalkylenoxid-haltigen Silikonderivaten der Formel 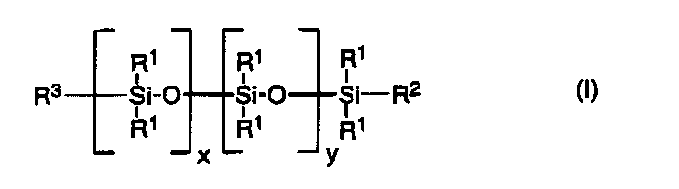
wobei:
R²=CH₃ oder 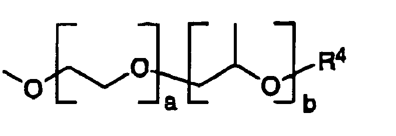 R³ = CH₃ oder R²
R⁴ = H, CH₃, 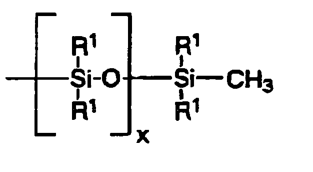 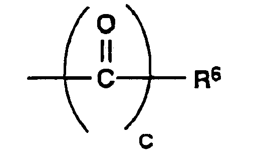
R⁶ ein organischer Rest aus 1 bis 40 Kohlenstoffatomen, der Amino-, Carbonsaure- oder Sulfonatgruppen enthalten kann oder, für den Fall c=O, auch das Anion einer anorganischen Säure bedeutet,
und wobei die Reste R¹ identisch oder unterschiedlich sein können, und ausgewählt sind aus der Gruppe Methyl, Ethyl, Propyl, Butyl, Isobutyl, Pentyl, Isopentyl, Hexyl, Octyl, Decyl. Dodecyl und Octadecyl, Cyclohexyl, aromatische Gruppen. Benzyl, Phenylethyl, Tolyl, Xylyl und R⁵, wobei: 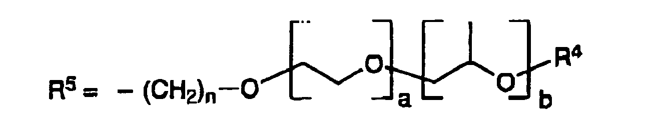 mit der Maßgabe, daß mindestens einer der Reste R¹, R² oder R³ ein polyalkylenoxidhaltiger Rest nach obengenannter Definition ist,
und n eine ganze Zahl von 1 bis 6 ist,
x und y ganze Zahlen derart sind, daß das Molekulargewicht des Polysiloxan-Blocks zwischen 300 und 30000 liegt,
a,b ganze Zahlen zwischen 0 und 50 sein können mit der Maßgabe, daß die Summe aus a und b größer als 0 ist, und c 0 oder 1 ist,
radikalisch polymerisiert, für kosmetische Formulierungen.

2. Verwendung nach Anspruch 1, **dadurch gekennzeichnet, daß** Formel I folgende Bedeutung besitzt:

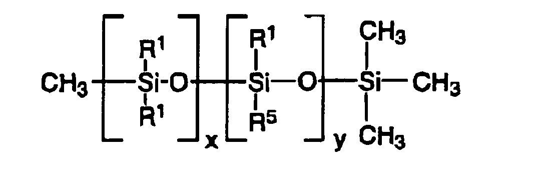

3. Verwendung nach Anspruch 1, **dadurch gekennzeichnet, daß** die Mengenverhältnisse
(a) 50-99,9 Gew.-% und
(b) 0,1-50 Gew.-% betragen.

4. Verwendung nach Anspruch 1-3 als Haarpflegemittel.

## Claims

1. The use of a polymer which is water-soluble or water-dispersible or which, if consisting of monomers with neutralizable radicals, is soluble in water or dispersible in water in neutralized form, which is obtainable by subjecting
(a) ethylenically unsaturated monomers to free-radicals polymerization in the presence of
(b) polyalkylene oxide-containing silicone derivatives of the formula 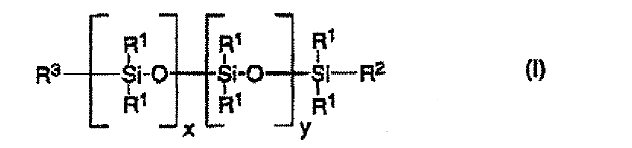
where:
R² = CH₃ or 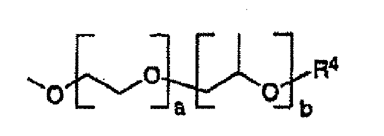 R³ = CH₃ or R²
R⁴ =H, CH₃, 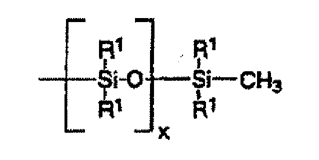 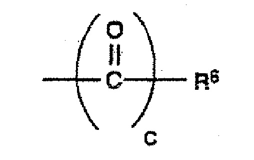
R⁶ is an organic radical of 1 to 40 carbons which may comprise amino, carboxyl or sulfonate groups, or, if c=0, is the anion of an inorganic acid,
and where the radicals R¹ can be identical or different and are selected from the group consisting of methyl, ethyl, propyl, butyl, isobutyl, pentyl, isopentyl, hexyl, octyl, decyl, dodecyl and octadecyl, cyclohexyl, aromatic groups, benzyl, phenylethyl, tolyl, xylyl and R⁵, where: 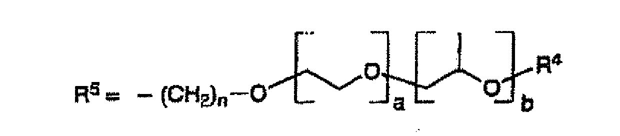 with the proviso that at least one of the radicals R¹, R² or R³ is a polyalkylene oxide-containing radical as defined above,
and n is an integer from 1 to 6,
x and y are integers such that the molecular weight of the polysiloxane block is from 300 to 30,000,
a,b can be integers from 0 to 50 with the proviso that the sum of a and b is greater than 0, and c is 0 or 1,
for cosmetic formulations.

2. The use according to claim 1, wherein formula I is:

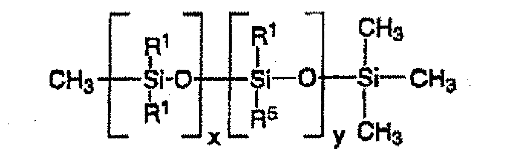

3. The use according to claim 1, wherein the proportions are
(a) 50-99.9 % by weight and
(b) 0.1-50 % by weight.

4. The use according to claims 1-3 as a haircare composition.

## Revendications

1. Utilisation de polymères qui sont solubles dans l'eau ou dispersables dans l'eau ou qui, dans le cas où ils sont constitués de monomères comportant des radicaux neutralisables, sont à l'état neutralisé solubles dans l'eau ou dispersables dans l'eau, pouvant être obtenus par polymérisation radicalaire
(a) de monomères à insaturation éthylénique en présence
(b) de dérivés de silicone contenant un polyoxyalkylène, de formule 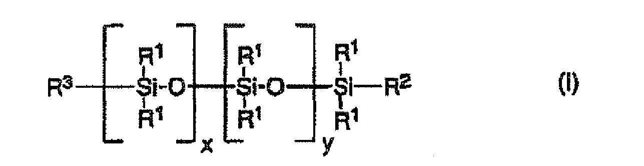
dans laquelle :
R² = CH₃ ou 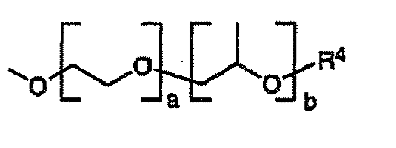 R³ = CH₃ ou R²
R⁴ = H, CH₃, 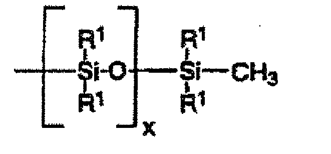 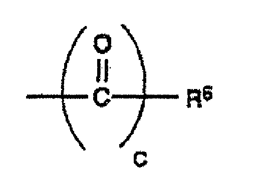
R⁶ représente un radical organique à base de 1 à 40 atomes de carbone, qui peut contenir des groupes amino, carboxy ou sulfonate ou, dans le cas où c = 0, également l'anion d'un acide inorganique,
et dans laquelle les radicaux R¹ peuvent être identiques ou différents et sont choisis dans l'ensemble constitué par les groupes méthyle, éthyle, propyle, butyle, isobutyle, pentyle, isopentyle, hexyle, octyle, décyle, dodécyle et octadécyle, cyclohexyle, les groupes aromatiques, benzyle, phényléthyle, tolyle, xylyle et R⁵, où : 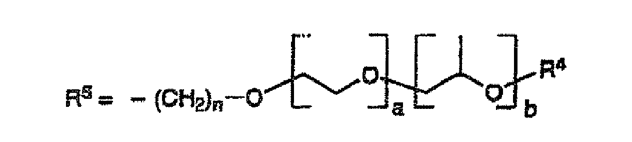 étant entendu qu'au moins l'un des radicaux R¹, R² et R³ est un radical contenant un groupe polyoxyalkylène selon la définition donnée plus haut,
et n est un nombre entier allant de 1 à 6,
x et y sont des nombres entiers tels que la masse moléculaire du bloc polysiloxane est comprise entre 300 et 30 000,
a, b peuvent être des nombres entiers compris entre 0 et 50, étant entendu que la somme de a et b est supérieure à 0 et
c est 0 ou 1,
pour des compositions cosmétiques.

2. Utilisation selon la revendication 1, **caractérisé en ce que** la formule I a la signification suivante :

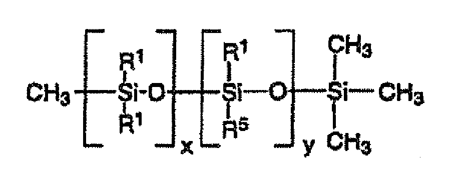

3. Utilisation selon la revendication 1, **caractérisé en ce que** les proportions sont les suivantes
(a) 50-99 % en poids et
(b) 0,1-50 % en poids.

4. Utilisation selon l'une quelconque des revendications 1 à 3, en tant qu'agent de soin capillaire.
